# FASCICULE DE BREVET EUROPEEN

(11) **EP 1 330 276 B1**
(45) Date de publication et mention de la délivrance du brevet: **20.02.2008**
(21) Numéro de dépôt: 01975939.8
(22) Date de dépôt: 19.10.2001
(51) Int. Cl.: A61M 5/142, A61M 5/168

(54) **DISPOSITIF MEDICAL IMPLANTABLE POUR DELIVRER UN LIQUIDE**
IMPLANTIERBARE MEDIZINISCHE VORRICHTUNG ZUM ABGEBEN EINER FLÜSSIGKEIT
IMPLANTABLE MEDICAL DEVICE FOR DELIVERING A LIQUID

(30) Priorité: 03.11.2000 CH 21462000
(43) Date de publication de la demande: 30.07.2003
(73) Titulaire: Allergan Medical S.A., 1024 Ecublens (CH)
(72) Inventeur: STERGIOPULOS, Nikolaus, CH-1025 St-Sulpice (CH)
(74) Mandataire: Besse, François
(86) Numéro de dépôt international: PCT/CH2001/000624
(87) Numéro de publication internationale: WO 2002/036184

(56) Documents cités:
- EP-A- 0 387 439
- US-A- 5 193 990
- US-A- 5 785 681

## Description

La présente invention se rapporte à un dispositif médical implantable pour l'administration de médicaments sous forme liquide.

L'état de la technique comprend plusieurs types de dispositifs implantables conçus pour l'administration de médicaments sous forme liquide. A titre illustratif, on peut citer les documents brevets suivants : EP-A-409511, US 4673391, WO 87/04629, WO 99/56803, US 5088983, EP-B-532561, EP-A-420620 et US 4299220.

Le plus souvent, ces dispositifs comprennent un réservoir destiné à contenir le liquide avant que celui-ci ne soit acheminé sur le site à traiter par le biais d'un conduit, un cathéter par exemple. Avec plusieurs dispositifs et en particulier avec ceux décrits chez US 5088983, WO 87/04629 ,US 4299220 et EP 0387439 A1, le réservoir est élastique ce qui rend possible, sous certaines conditions de pression régnant à l'intérieur et/ou l'extérieur du réservoir, l'expulsion passive du liquide contenu à l'intérieur du réservoir.

Afin d'améliorer la régulation du débit du liquide expulsé dans de telles conditions, divers régulateurs de flux ont été développés.

Le dispositif décrit dans US 4299220 se caractérise par un régulateur de débit qui comprend une chambre de volume variable constituée d'une membrane faisant office de valve et un conduit reliant le réservoir à la chambre de volume variable, le conduit comprenant un ou plusieurs limiteurs de débit dont la longueur est choisie en fonction du débit que l'on souhaite instaurer. Suivant les conditions de pression prédéfinies dans la chambre de volume variable la valve constituée par la membrane laisse ou empêche le passage de liquide en direction du site à traiter.

Avec les dispositifs de l'état de la technique tels que décrits précédemment, il est toujours nécessaire, pour définir la quantité de médicament à administrer, de recourir à une valve ou un système similaire que l'on place en aval du régulateur de débit. La précision du volume de médicament à administrer dépend donc principalement de la précision du contrôle de la valve ou du système similaire. Un contrôle parfait ne peut cependant être assuré avec les dispositifs de l'état de la technique, l'incertitude qui subsiste à ce niveau implique que le volume de médicament à administrer ne peut être fixé avec une précision satisfaisante. Ce problème est particulièrement important pour des thérapies qui exigent l'administration d'un volume très précis de médicaments.

La présente invention vise entre autres à remédier à cet inconvénient en proposant un dispositif offrant un contrôle amélioré du volume de liquide à administrer vers la zone à traiter. Elle se rapporte à un dispositif implantable pour l'administration de médicaments sous forme liquide comprenant :
- un réservoir muni d'un orifice d'entrée et d'un orifice de sortie, ledit réservoir étant adapté pour expulser du liquide,
- une chambre de volume variable munie d'un orifice d'entrée et d'un orifice de sortie , le volume de la chambre de volume variable étant notablement inférieur à celui du réservoir .
- un premier conduit reliant l'orifice de sortie du réservoir avec l'orifice d'entrée de la chambre de volume variable de façon à assurer un remplissage de cette dernière, ledit premier conduit comprenant des moyens d'interruption et d'ouverture de l'écoulement du liquide,
- un deuxième conduit dont l'une des extrémités est connectée sur l'orifice de sortie de la chambre de volume variable,

L'originalité de l'invention réside essentiellement dans le fait que :
- ledit deuxième conduit est dépourvu de valve ou de tout autre dispositif similaire permettant de déterminer le volume à administrer,
- ladite chambre de volume variable est adaptée pour expulser un volume prédéfini de liquide à partir du moment ou son expansion a atteint une valeur déterminée.

Comme on pourra le constater par la suite et contrairement aux dispositifs de l'état de la technique, dans le cadre de la présente invention, la détermination du volume à administrer n'est pas dépendante d'une valve ou de moyens similaires placés en aval de la chambre de volume variable.

Le dispositif selon la présente invention se différencie des dispositifs de l'état de la technique notamment par le fait que l'administration du médicament est réalisée par une succession de petits volumes Vₑₓₚ. Par « petit volume », il faut comprendre un volume très inférieur à celui du réservoir, de préférence au moins 5 fois inférieur.

Selon un autre mode de réalisation de l'invention, le mouvement de la chambre de volume variable est maintenu entre deux valeurs V_{inf} et Vₛᵤₚ au moyen de limiteurs. Dans une telle configuration, le volume expulsé Vₑₓₚ est alors égal à Vₛᵤₚ - V_{inf}.

Dans un autre mode de réalisation, la position des limiteurs peut être variée, ce qui permet pour un même dispositif de choisir le volume expulsé Vₑₓₚ qui convient le mieux pour le traitement.

L'invention sera décrite ci-après au moyen des figures suivantes :
La figure 1 représente un mode de réalisation du dispositif selon l'invention
La figure 2 est un graphique qui présente le débit du liquide en fonction du temps

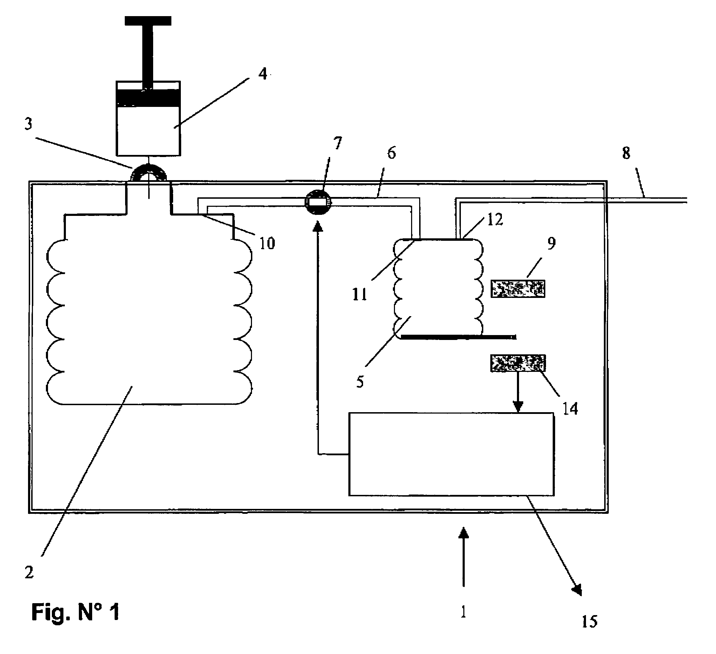

Le dispositif implantable (1) illustré à la figure 1 comprend un réservoir élastique (2) en forme de soufflet destiné à recevoir le médicament sous forme liquide qui est introduit à travers un orifice d'entrée (3). Ce dernier peut être constitué d'un septum, le médicament étant alors introduit au moyen d'une seringue (4) dont l'aiguille perce le septum.

Le réservoir est relié à une chambre de volume variable (5) par l'intermédiaire d'un conduit (6) muni d'une valve (7). La chambre de volume variable (5), dont le volume est considérablement plus faible que celui du réservoir (2), est également en forme de soufflet. Un deuxième conduit (8) vient se connecter par l'une de ses extrémités à l'orifice de sortie (12) de la chambre de volume variable, l'autre extrémité du deuxième conduit (8) aboutissant vers le site à traiter.

Deux limiteurs (9,14) sont placés de façon à limiter la variation de volume de la chambre de volume variable (5) entre un volume inférieur V_{inf}, grâce au limiteur inférieur (9) et un volume supérieur Vₛᵤₚ, grâce au limiteur supérieur (14).

La valve (7) placée sur le premier conduit (6) peut être activée de différentes manière. De préférence, elle doit se refermer lorsque l'expansion de la chambre de volume variable (5) a atteint une valeur déterminée. Cette activation peut être réalisée électroniquement ou mécaniquement. A cet effet, on peut par exemple prévoir qu'un senseur situé sur la surface de limiteur (14) envoie un signal à une boîte de contrôle (15) qui ensuite déclenche le mécanisme de fermeture de la valve (7) lorsque la chambre de volume variable a atteint un certain volume appelé volume supérieur Vₛᵤₚ. La boîte de contrôle (15) peut également exercer une fonction télémétrique pour la réception de signaux télémétriques (par radiofréquence ou autres) qui sont envoyés de l'extérieur et qui servent à transmettre les paramètres de contrôle et de fonctionnement de l'appareil. De tels paramètres sont, par exemple, la période du cycle d'ouverture/fermeture de la valve (7) et la position des limiteurs (9,14).

Le principe de fonctionnement du dispositif décrit à la figure 1 est le suivant :

Dans une première étape, le réservoir élastique (2) est rempli au moyen d'une seringue (4), la valve (7) étant fermée. A partir d'un certain degré d'extension du réservoir élastique (2), la force de rappel de celui-ci est tel que le liquide peut être expulsé dans le conduit (6).

Lorsque la valve (7) est ouverte, le liquide est acheminé vers la chambre de volume variable (5). Dès que le volume supérieur Vₛᵤₚ de la chambre de volume variable est atteint, la valve (7) se ferme et le liquide est acheminé par la force de rappel de la chambre de volume variable (5) dans le deuxième conduit (8) vers le site à traiter.

Lorsque la chambre de volume variable (5) se retrouve à son niveau de volume inférieur V_{inf}, il n'y a plus d'expulsion du liquide en dehors de celle-ci. Au bout d'un intervalle de temps Δt, déterminé en fonction de la posologie souhaitée, la valve (7) s'ouvre à nouveau et le processus décrit précédemment se reproduit. L'administration du médicament est donc réalisée par une succession d'expulsions de doses dont le volume Vₑₓₚ = Vₛᵤₚ - V_{inf} est connu de façon précise. Il en résulte que la quantité totale de médicament à administrer peut être déterminée très précisément grâce au dispositif de l'invention.

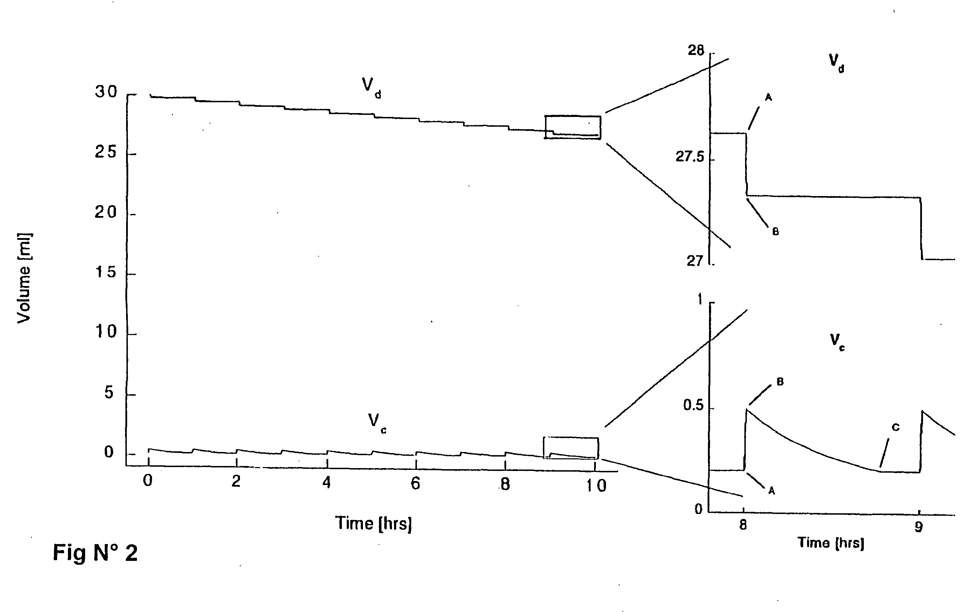

La figure 2 représente un exemple graphique du fonctionnement de l'appareil. La partie de gauche du graphe montre la variation du volume du réservoir élastique (2) V_{d} et celle du volume de la chambre de volume variable (5) V_{c} pendant 10 cycles de fonctionnement de l'appareil. Dans cet exemple, la période de chaque cycle est d'une heure, le volume initial duréservoir élastique (2) V_{d} est de 30 ml et s'abaisse à 5 ml une fois que tout le liquide est expulsé, les volumes supérieur Vₛᵤₚ et inférieur V_{inf} de la chambre de volume variable (5) sont respectivement de 0.5 ml et de 0.2 ml. Le volume expulsé Vₑₓₚ de la chambre de volume variable dans chaque cycle de fonctionnement est donc de 0.3 ml, ce qui donne un débit moyen de liquide expulsé de Vₑₓₚ/T , soit 0.3 ml/heure. La compliance du réservoir élastique (2) est de 0.1 ml/mmHG alors que celle de la chambre de volume variable (5) est de 0.02 ml/mmHG. La résistance à l'entrée de la chambre de volume variable (5) est de 20000 mmHg/(ml/s) alors que la résistance de sortie se monte à 100000 mmHg/(ml/s).

En somme, les paramètres doivent être choisis de façon à ce que la pression soit toujours plus élevée dans le réservoir élastique (2) que dans celle de la chambre de volume variable (5), ceci afin d'assurer un remplissage et une pression suffisantes dans la chambre de volume variable (5) pour permettre une expulsion du liquide en dehors de celle-ci.

La partie droite de la figure 2 montre deux agrandissements de sa partie gauche, soit la variation des volumes V_{d} et V_{c} pendant 1 cycle de fonctionnement. Les points A et B correspondent au moment de l'ouverture et fermeture de la valve (7). Le point C correspond au moment où l'expulsion du liquide par la chambre de volume variable (5) est interrompue par le limiteur (9).

Il convient de relever que lors du remplissage de la chambre de volume variable (5), une quantité infime de liquide peut directement passer dans le deuxième conduit (8), de sorte qu'à chaque cycle, outre le volume expulsé Vₑₓₚ, un volume supplémentaire est administré. Ce volume supplémentaire est minime si la phase de remplissage de la chambre de volume variable (5) est très courte par rapport à la phase de l'expulsion du liquide (càd le temps entre les points A et B est très inférieur au temps entre les points B et C. Dans certains cas, il peut s'avérer utile de minimiser ce volume supplémentaire, voire de le supprimer, afin de déterminer le plus précisément possible la quantité de médicament à administrer. Ceci peut être réalisé en utilisant un deuxième conduit (8) dont la résistance à l'écoulement du liquide est notablement supérieure à celle du premier conduit (6). Alternativement ou en outre, on peut prévoir de placer une valve de type ON/OFF sur le deuxième conduit (8), cette dernière se fermant lorsque la valve (7) du premier conduit (6) s'ouvre.

Il convient de souligner qu'un volume expulsé Vₑₓₚ vers le site à traiter est connu avec un très haut degré de précision. En effet, sa détermination est pratiquée par une méthode statique, soit la mesure de la différence entre le volume supérieur Vₛᵤₚ et le volume inférieur V_{inf}, ces deux volumes étant indirectement connus par la position des limiteurs (9,14).

Dans le cas où le dispositif ne contiendrait pas de limiteurs, le volume expulsé peut être déterminé en usine.

Outre le fait que Vₑₓₚ peut être déterminé très précisément, la valeur Δt est déterminée avec un haut degré de précision. Il devient dès lors possible de prévoir la quantité exacte de médicament qui est réellement administrée ainsi que le débit moyen d'administration. Inversement, dans les dispositifs de l'état de la technique, la détermination du volume expulsé est pratiquée par mesure dynamique, c'est-à-dire en fonction du débit et du temps d'ouverture de la valve. La connaissance du volume expulsé ne peut être que plus imprécise dans ce cas.

En principe, le débit moyen du liquide expulsé est égal à Vₑₓₚ/T, cette valeur étant elle-même égale à la variation de volume du réservoir (2), V_{d}, par rapport au temps. Il est donc possible d'utiliser un senseur qui mesure avec grande précision le volume V_{d} en tout temps afin d'effectuer un contrôle de sécurité du débit moyen.

A noter que l'invention ne se limite pas à des réservoirs élastiques qui expulsent le liquide de manière passive. On peut parfaitement concevoir un système où le réservoir est placé dans une cavité remplie d'un gaz et que l'élévation de pression du gaz dans la cavité réduit le volume du réservoir pour faire expulser le liquide. Il n'est d'ailleurs pas nécessaire de recourir à un réservoir élastique, tout réservoir implantable muni d'un système d'expulsion du liquide peut être envisagé.

De même, la chambre de volume variable n'a pas nécessairement la forme d'un soufflet. On peut par exemple utiliser un ballonnet dont la compliance est élevée lors de son remplissage et quasi nulle lorsque celui-ci est rempli.

A relever enfin que le nombre de réservoirs élastiques utilisés avec le dispositif selon la présente invention n'est pas limité. Il est parfaitement envisageable d'utiliser au moins deux réservoirs contenant chacun un médicament différent, l'ouverture de chaque réservoir se produisant successivement et/ou en alternance ou simultanément si les deux médicaments doivent être mélangés préalablement à leur administration.

Avec une telle configuration, une seule valve multiple peut être utilisée, chaque réservoir élastique venant se connecter à ladite valve multiple.

## Revendications

1. Dispositif implantable (1) pour l'administration de médicaments sous forme liquide comprenant :
- un réservoir (2) muni d'un orifice d'entrée (3) et d'un orifice de sortie (10), ledit réservoir (2) étant adapté pour expulser du liquide.
- une chambre de volume variable (5) munie d'un orifice d'entrée (11) et d'un orifice de sortie (12), le volume de la chambre de volume variable (5) étant notablement inférieur à celui du réservoir (2),
- un premier conduit (6) reliant l'orifice de sortie (10) du réservoir (2) avec l'orifice d'entrée (11) de la chambre de volume variable (5) de façon à assurer un remplissage de cette dernière, ledit premier conduit (6) comprenant des moyens d'interruption et d'ouverture de l'écoulement du liquide (7),
- un deuxième conduit (8) dont l'une des extrémités est connectée sur l'orifice de sortie (12) de la chambre de volume variable (5),
**caractérisé par le fait que** :
- ledit deuxième conduit (8) est dépourvu de valve ou de tout autre dispositif similaire permettant de déterminer le volume à administrer,
- ladite chambre de volume variable (5) est adaptée pour expulser un volume prédéfini (Vₑₓₚ) de liquide à partir du moment ou son expansion a atteint une valeur déterminée.

2. Dispositif selon la revendication précédente, **caractérisé par le fait que** les moyens d'interruption (7) sont structurellement adaptés pour être activés à l'instant où l'expansion de la chambre de volume variable (5) a atteint une valeur déterminée.

3. Dispositif selon la revendication 1 ou 2, **caractérisé par le fait que** les moyens d'ouverture (7) sont structurellement adaptés pour être activés à intervalles de temps réguliers (Δt) de sorte que l'acheminement du liquide hors de la chambre de volume variable est réalisé par une succession d'expulsions de volumes prédéfinis (Vₑₓₚ).

4. Dispositif selon l'une quelconque des revendications précédentes, **caractérisé par le fait que** la résistance à l'écoulement du fluide dans le deuxième conduit (8) est notablement supérieure à celle du premier conduit (6).

5. Dispositif selon l'une quelconque des revendications précédentes, **caractérisé par le fait que** le deuxième conduit (8) comprend des moyens d'interruption du liquide du type ON/OFF qui sont conçus de manière à stopper l'écoulement du liquide lorsque les moyens d'ouverture (7) sont activés.

6. Dispositif selon l'une quelconque des revendications précédentes, **caractérisé par le fait que** le volume du réservoir (2) est au moins 5 fois supérieur à celui de la chambre (5).

7. Dispositif selon l'une quelconque des revendications précédentes, **caractérisé par le fait qu'**il comprend des limiteurs de volume (9,14) destinés à définir le volume expulsé (Vₑₓₚ).

8. Dispositif selon la revendication 6, **caractérisé par le fait que** la position des limiteurs (9,14) est variable.

## Claims

1. Implantable device (1) for administering medicines in liquid form, comprising:
- a reservoir (2) provided with an inlet (3) and an outlet (10), said reservoir (2) being adapted to expel the liquid,
- a variable-volume chamber (5) provided with an inlet (11) and an outlet (12), the volume of the variable-volume chamber (5) being notably smaller than that of the reservoir (2),
- a first conduit (6) connecting the outlet (10) of the reservoir (2) to the inlet (11) of the variable-volume chamber (5) in order to fill the latter, said first conduit (6) comprising means (7) for interrupting and opening the flow of the liquid,
- a second conduit (8), one of whose ends is connected to the outlet (12) of the variable-volume chamber (5),
**characterized in that**:
- said second conduit (8) is without a valve or any other similar device allowing the volume to be administered to be specified
- said variable-volume chamber (5) is adapted to expel a predefined volume (Vₑₓₚ) of liquid from the moment when its expansion has reached a specified value.

2. Device according to the preceding claim, **characterized in that** the interrupting means (7) are structurally adapted to be activated at the instant when the expansion of the variable-volume chamber (5) has reached a specified value.

3. Device according to Claim 1 or 2, **characterized in that** the opening means (7) are structurally adapted to be activated at regular time intervals (Δt) in such a way that the delivery of the liquid from the variable-volume chamber is effected in a succession of expulsions of predefined volumes (Vₑₓₚ).

4. Device according to any one of the preceding claims, **characterized in that** the resistance to the flow of the fluid in the second conduit (8) is notably greater than that of the first conduit (6).

5. Device according to any one of the preceding claims, **characterized in that** the second conduit (8) comprises means of the ON/OFF type for interrupting the liquid, which means are designed in such a way as to stop the flow of the liquid when the opening means (7) are activated.

6. Device according to any one of the preceding claims, **characterized in that** the volume of the reservoir (2) is at least 5 times greater than that of the chamber (5).

7. Device according to any one of the preceding claims, **characterized in that** it comprises volume limiters (9, 14) intended to define the expelled volume (Vₑₓₚ).

8. Device according to claim 6, **characterized in that** the position of the limiters (9, 14) is variable.

## Patentansprüche

1. Implantierbare Vorrichtung (1) zur Verabreichung von Medikamenten in flüssiger Form, die aufweist:
- ein Reservoir (2), das mit einer Eingangsöffnung (3) und mit einer Ausgangsöffnung (10) versehen ist, wobei das Reservoir (2) geeignet ist, um Flüssigkeit auszustoßen,
- eine Kammer mit variablem Volumen (5), die mit einer Eingangsöffnung (11) und mit einer Ausgangsöffnung (12) versehen ist, wobei das Volumen der Kammer mit variablem Volumen (5) deutlich kleiner ist als dasjenige des Reservoirs (2),
- eine erste Leitung (6), die die Ausgangsöffnung (10) des Reservoirs (2) mit der Eingangsöffnung (11) der Kammer mit variablem Volumen (5) verbindet, um ein Füllen dieser letzteren zu gewährleisten, wobei die erste Leitung (6) Unterbrechungs- und Öffnungsmittel für das Fließen der Flüssigkeit (7) aufweist,
- eine zweite Leitung (8), deren eines Ende mit der Ausgangsöffnung (12) der Kammer mit variablem Volumen (5) verbunden ist,
**dadurch gekennzeichnet, dass**:
- die zweite Leitung (8) kein Ventil oder eine andere ähnliche Vorrichtung aufweist, die es ermöglicht, das zu verabreichende Volumen zu bestimmen,
- die Kammer mit variablem Volumen (5) geeignet ist, um ein vordefiniertes Volumen (Vₑₓₚ) an Flüssigkeit ab dem Moment auszustoßen, in dem ihre Ausdehnung einen bestimmten Wert erreicht hat.

2. Vorrichtung nach dem vorhergehenden Anspruch, **dadurch gekennzeichnet, dass** die Unterbrechungsmittel (7) strukturell geeignet sind, um in dem Moment aktiviert zu werden, in dem die Ausdehnung der Kammer mit variablem Volumen (5) einen bestimmten Wert erreicht hat.

3. Vorrichtung nach Anspruch 1 oder 2, **dadurch gekennzeichnet, dass** die Öffnungsmittel (7) strukturell geeignet sind, um in regelmäßigen Zeitintervallen (Δt) aktiviert zu werden, so dass der Transport der Flüssigkeit aus der Kammer mit variablem Volumen durch eine Folge von Ausstößen mit vordefinierten Volumen (Vₑₓₚ) erfolgt.

4. Vorrichtung nach einem der vorhergehenden Ansprüche, **dadurch gekennzeichnet, dass** der Widerstand gegen das Fließen des Fluids in der zweiten Leitung (8) deutlich größer ist als derjenige der ersten Leitung (6).

5. Vorrichtung nach einem der vorhergehenden Ansprüche, **dadurch gekennzeichnet, dass** die zweite Leitung (8) Unterbrechungsmittel der Flüssigkeit vom Typ ON/OFF aufweist, die so konzipiert sind, dass sie das Fließen der Flüssigkeit anhalten, wenn die Öffnungsmittel (7) aktiviert werden.

6. Vorrichtung nach einem der vorhergehenden Ansprüche, **dadurch gekennzeichnet, dass** das Volumen des Reservoirs (2) mindestens 5 mal größer ist als dasjenige der Kammer (5).

7. Vorrichtung nach einem der vorhergehenden Ansprüche, **dadurch gekennzeichnet, dass** sie Volumenbegrenzer (9, 14) aufweist, die dazu bestimmt sind, das ausgestoßene Volumen (Vₑₓₚ) zu definieren.

8. Vorrichtung nach Anspruch 6, **dadurch gekennzeichnet, dass** die Stellung der Begrenzer (9, 14) variabel ist.
